Europäisches Patentamt

European Patent Office

Office européen des brevets

(11) Publication number: **0 343 819**
**A1**

## (12) EUROPEAN PATENT APPLICATION

(21) Application number: **89304856.1**

(22) Date of filing: **12.05.89**

(51) Int. Cl.⁴: **C07C 29/16 , C07C 31/125**

(30) Priority: **16.05.88 GB 8811576**

(43) Date of publication of application:
**29.11.89 Bulletin 89/48**

(84) Designated Contracting States:
**AT BE CH DE ES FR GB IT LI NL SE**

(71) Applicant: **EXXON CHEMICAL PATENTS INC.**
**200 Park Avenue**
**Florham Park New Jersey 07932(US)**

(72) Inventor: **Hanin, Jean Alexandre Andre**
**19 Avenue des Tourterelles**
**B-1330 Rixensart(BE)**

(74) Representative: **Bawden, Peter Charles et al**
**EXXON CHEMICAL LIMITED EXXON**
**CHEMICAL TECHNOLOGY CENTRE PO Box 1**
**Abingdon Oxfordshire OX13 6BB(GB)**

(54) **Production of alcohols.**

(57) In the oxo process for alcohol production water is injected into the hydrofinishing stage to reduce heavies and carbonyl and also permit use of sulphur tolerant catalyst in hydrogenation and/or hydrofinishing and thus allow use of sulphur containing olefine feeds.

EP 0 343 819 A1

## PRODUCTION OF ALCOHOLS

This invention relates to the production of alcohols by hydroformylation processes and in particular the production of alcohols of high purity by a simplified process.

The hydroformylation process, in general terms, is a process involving the preparation of oxygenated organic compounds by the reaction of carbon monoxide and hydrogen (synthesis gas) with carbon compounds containing olefinic unsaturation. The reaction is performed under hydroformylation conditions in the presence of a carbonylation catalyst or catalyst precursor such as dicobaltoctacarbonyl, and results in the formation of a compound eg an aldehyde which has one more carbon atom in its molecular structure than the feedstock. Subsequent hydrogenation of the primary product leads to higher alcohols which may be used for example for conversion into plasticizers.

Typically in higher alcohol production the feedstock for a hydroformylation process is a commercial $C_6$-$C_{12}$ olefine fraction and the desired end product is the respective $C_7$-$C_{13}$ saturated alcohol or derived mixed alcohol product, produced by hydrogenation of the aldehyde oxonation product. By virtue of the nature of the feedstock commonly available to industry, and indeed of the catalyst and reaction parameters employed, the oxonation reaction yields a range of products due to the numerous secondary reactions which take place. The main commercial products of the hydroformylation reaction are aldehydes and alcohols, with side reactions in the oxonation, demetalling and hydrogenation sections of the process system producing some 5 to 20 wt.% of high boiling materials by condensation, esterification and dehydration reactions.

In a conventional higher oxo alcohol process, the feedstock as described above is fed together with synthesis gas into an oxonation unit where catalytic hydroformylation takes place using e.g. hydrocobaltoctacarbonyl as the active catalyst species. The oxonation unit product passes to a unit for removing catalyst, and then to a hydrogenation unit where it is hydrogenated to form the desired higher alcohol; the catalyst used in the hydrogenation unit are typically sulphur sensitive catalysts such as reduced nickel and cuprous/chromium which suffer from the disadvantage that they cannot be regenerated. It is therefore necessary to use feeds which are substantially free of sulphur to achieve acceptable catalyst life.

The product mixture after hydrogenation comprising the higher alcohol, the high boiling materials mentioned above and a low boiling fraction is then passed to a distillation unit where low boiling materials, high boiling materials and the desired alcohol product are separated. The low boiling material passing off contains overhead olefine feed and paraffins. The high boiling material usually contains dimers such as ethers and ether-alcohols (e.g. $C_{20}$ compounds in $C_{10}$ alcohol production) and trimers such as acetals (e.g. $C_{30}$ compounds in $C_{10}$ alcohol production) and heavier; although substantially alcohol free (apart from the heavy ether-alcohols), it may contain a minor amount of alcohol which has not been removed in the distillation stage where the higher alcohol product of the hydroformylation process is separated. In our European patent publication 0183545 we describe a process for upgrading these heavy fractions to more useful alcohol.

The fraction containing the desired alcohol generally contains an undesirably high proportion of compounds containing carbonyl groups. A widespread use for such alcohols is in the production of plasticiser esters where the presence of even low levels of carbonyl compound can result in undesirable colour and odour in the plasticiser and plasticised materials, generally an acceptable product should have a carbonyl content no greater than 0.15 Mg/KOH/Gram and a heavies (products with a carbon number of 2 or more times that of the olefine - typically dimers or trimers) content lower 0.1 wt%. These products are therefore frequently subjected to a hydrofinishing process to reduce the ultimate carbonyl content in the alcohol to acceptable levels. Nickel catalysts or supported nickel catalysts are normally used in this hydrofinishing since they are highly selective and do not catalyse the formation of large amounts of undesirable heavy products and are reactive at sufficiently low temperatures to avoid decomposition or dehydration of the desirable alcohols. The need to use these catalysts however has the disadvantage that they are expensive, cannot be regenerated and are highly sensitive to the presence of sulphur; in the product. This is another reason why it is necessary to use feeds that are substantially free of sulphur.

We have now developed a hydroformylation process for the production of alcohols which further reduces the heavy content of the alcohol and can allow the use of sulphur insensitive catalysts in the hydrogenation and/or hydrofinishing stages.

The present invention therefore provides a process for the production of alcohols comprising
    (i) hydroformylation of an olefine
    (ii) catalytic hydrogenation of the hydroformylation product
    (iii) separation of an alcohol containing fraction from the product of catalytic hydrogeneration and

2

(iv) subjecting the alcohol containing fraction to catalytic hydrofinishing

the improvement in which comprises introducing water into the catalytic hydrofinishing stage of the process.

We have found that the incorporation of water in the hydrofinishing stage allows a wider range of catalysts to be used in both the hydrogenation and hydrofinishing stages of the process and can also allow higher sulphur levels to be tolerated in the olefine feed than have hitherto been economically acceptable.

The process is applicable to the production of alcohols from any olefines which may be subjected to hydroformylation but is particularly suited to the hydroformylation of $C_6$ to $C_{12}$ olefines for the production of $C_7$-$C_{13}$ alcohols.

Conventional hydroformylation conditions may be used in the process of this invention the catalyst may be for example cobalt based and the operating temperatures, pressures and other conditions such as synthesis gas composition may be controlled in accordance with the usual expertise of the person skilled in the art to maximise yield of the desired higher alcohol product. For example the hydroformylation reaction may be carried out at a pressure of 15-30 Megapascals, and a temperature of from 120-190°C.

The catalyst may be used as desired in active form, for example in a concentration of from 0.05-3 wt% preferably 0.05 and 1 wt% as metal based on the olefinic feed. Typically the synthesis gas used might have a $H_2$:CO volume ratio in the range 0.9:1-1.5:1. The catalyst is generally separated from the product mixture prior to the hydrogenation step.

The catalytic hydrogenation step (ii) of the process of this invention is preferably carried out at a temperature in the range 150°C to 240°C preferably 170°C to 200°C. The preferred pressure of operation is in the 4.5-6.5 Megapascals range. It has been found that the hydrogenation reaction to the desired alcohol enriched product mixture proceeds satisfactorily at a space velocity of from 0.2-2 vol/vol/hour, with the more preferred space velocity range being from 0.75-1.25 vol/vol/hour. By space velocity is meant the hourly flow by volume of the liquid reactants per unit volume of catalyst employed.

The traditional hydrogenation catalysts may be employed such as the so called copper chrome (also termed Cu/Cr or copper-chromium oxide or copper-chromite) catalysts and supported nickel; the present invention however enables sulphur resistant regenerable catalysts such as sulphided trimetallic and trimetallic catalysts in particular sulphided cobalt/molybdenum, sulphided nickel/molybdenum, sulphided nickel/tungsten and their derivatives to be used in the hydrogenation step of the process and their use is an aspect of the present invention.

The product of the hydrogenation step is generally distilled to separate the desired alcohol fraction from the light fraction and the heavy products which may, if desired, be further treated to produce more alcohol by for example the techniques described in European Patent Publication 0183545.

The desired alcohol fraction typically contains up to 2 weight % carbonyl containing compounds particularly aldehydes and such an amount leads to poor colour and undesirable odours in plasticisers produced from the alcohols. The alcohol fraction is therefore subjected to hydrofinishing to reduce the carbonyl level. Conventional hydrofinishing uses nickel catalysts generally at 70 to 110°C, such a sensitive catalyst being required to allow sufficiently low temperatures to be used to prevent formation of light materials by decomposition or by dehydration of the alcohol and prevent formation of "heavies" by the reaction of the alcohol with aldehyde. The life of these catalysts is unacceptably short if trace amounts of sulphur are present and thus the use of sulphur free olefine feed is needed. Due to their generally lower activity higher temperatures would however be required if sulphur resistant catalysts such as cobalt/molybdenum, nickel/tungsten or nickel/molybdenum were used which would result in a deterioration in alcohol quality and at these higher temperatures lights and heavies formation increases.

The provision of water in the hydrofinishing step according to the present invention has been found to reduce the carbonyl and heavies content in the final product when using the traditional hydrofinishing catalysts and furthermore allows the use of sulphur resistant catalysts in the hydrofinishing step of the process such as the sulphided cobalt/molydenum, nickel/tungsten or nickel/molybdenum described above and this use is another feature of the present invention. In addition the injection of water into the hydrofinishing reaction can allow the use of sulphur resistant catalysts in the hydrogenation stage of the process which in turn enables feeds containing as much as 1000 ppm of sulphur to be used. It is however preferred that the feed contain no more than 200 ppm of sulphur. We have found that injection of from 0.1 to 1.5 volume % of water based on the volume of feed to the hydrofinishing section is sufficient to achieve the benefits of the present invention.

It is preferred that the water be removed from the final alcohol after the hydrofinishing step preferably by distillation, nitrogen stripping or flash under vacuum. The water may be recycled to the hydrofinishing stage of the reaction.

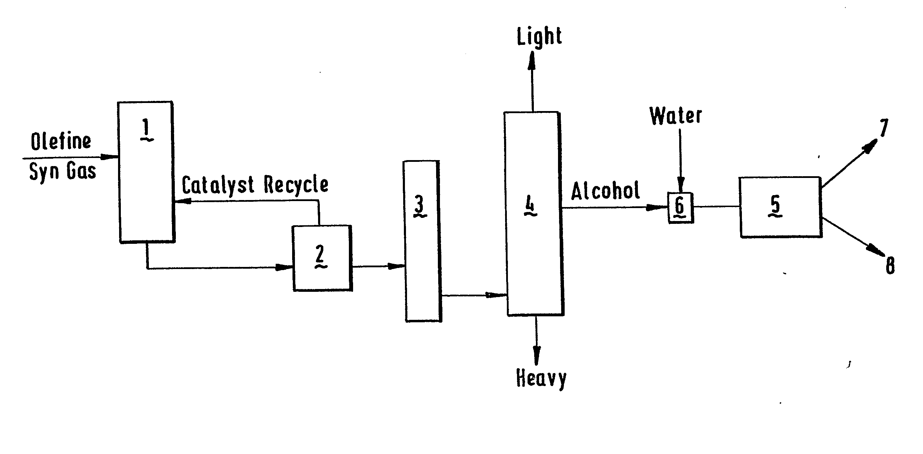

The process of the present invention is schematically illustrated by reference to the accompanying drawing in which olefine feed and syngas are fed to the hydroformylation reactor 1 where they undergo hydroformylation. The crude hydroformylation product then passes to the catalyst recovery cycle 2 where hydroformylation catalyst is recovered and recycled to the hydroformylation reactor. The crude product free of catalyst then passes to the hydrogenation reactor 3 where it is hydrogenated to convert aldehydes to alcohols etc. The hydrogenated product then passes to the distillation towers 4 where the light and heavy fractions are separated from the desired alcohol stream, the light and heavy fractions may be further processed as desired. The alcohol stream then passes to the hydrofinishing reactor 5 and the desired quantity of water is injected at 6 (or in the hydrofinishing reactor), after hydrofinishing the water is removed at 7 and the alcohol obtained at 8.

Example 1

A typical hydroformylation process was used to produce higher alcohol from a substantially sulphur free feed comprising (i) syn gas containing hydrogen and carbon monoxide in a molar ratio of 1.16:1 and (ii) a commercially available stream of branched nonenes including about 2 wt % octenes and about 8 wt % decenes. The olefin feed was delivered at a rate of 1.5 1/hr (1115 g/hr), and the syn gas at a rate of 640 standard 1/hr, into three 1.0 litre capacity oxonation reactors arranged in series, and the reaction was carried out at a pressure of 30 Megapascals and a temperature of 175°C, using a cobalt catalyst at 0.3 wt % based on the feed. The resultant crude oxo product containing higher aldehyde was decobalted to less than 10 ppm cobalt in conventional manner by neutralizing the cobalt hydrocarbonyl with sodium hydroxide and washing with water, and thereafter was fed to a conventional hydrogenation train where, using Cu/Cr and Ni catalysts, a hydrogen pressure of 5 Megapascals and a temperature between 150-190°C a product mixture containing the desired higher alcohol was formed. This product mixture was then distilled under vacuum to produce three fractions, a light oxo fraction (LOF), a higher alcohol fraction (HA) and a bottoms product of heavy oxo fraction (HOF).

Six higher alcohol fractions were obtained from this process using slightly different conditions and passed to a hydrofinishing reactor reactor composed of four tubes in series equipped with intermediate sample points. The operating conditions were pressure 5.5 Megapascals, space velocity 1.5 volume of alcohol fraction per total volume of catalyst per hour, hydrogen 60 normal volume per volume of alcohol fraction, at a temperature indicated in Table 1 with and without water injection as set out in table 1. Taking samples after each tube and analysing for carbonyl number allows one to follow the carbonyl reduction as a function of the residence ;time or the inverse of the space velocity.

Tests were carried out using various catalysts in the hydrofinishing reactor and the results are set out in Table 1 showing that when using the conventional supported reduced nickel catalysts (catalysts 1,2 and 3) the level of both carbonyl and heavy products in the alcohol is substantially reduced when water is present in the hydrofinishing reactor. The examples also show that the process may be applied using the sulphided nickel/molybdenum sulphur resistant catalysts (4 and 5) at the higher temperatures at which satisfactory product cannot be achieved without water addition.

TABLE 1

| CATALYST | 1 | | 1 | | 1 | | 1 | | 2 | | 2 | |
|---|---|---|---|---|---|---|---|---|---|---|---|---|
| Temperature °C | 90°C | | 90°C | | 110°C | | 110°C | | 110°C | | 110°C | |
| Water vol.% | 0 | | 0.3 | | 0 | | 0.6 | | 0 | | 1.1 | |
| Residence time hrs | CN | HY | CN | HY | CN | HY | CN | HY | CN | HY | CN | HY |
| 0.0 | 6.12 | 0 | 6.28 | 0.0 | 6.05 | 0 | 6.95 | 0 | 3.48 | 0 | 3.3 | 0 |
| 0.16 | 2.48 | | 2.57 | | 1.43 | | 1.20 | | | | 0.67 | |
| 0.33 | 0.32 | | 0.23 | | 0.3 | | 0.28 | | 1.11 | | 0.08 | |
| 0.5 | 0.22 | | 0.100 | | 0.27 | | 0.14 | | 0.39 | | 0.07 | |
| 0.66 | 0.19 | 0.04 | 0.07 | 0.0 | 0.22 | 0.46 | 0.05 | 0.03 | 0.36 | 0.17 | | 0.00 |

| CATALYST | 4 | | 4 | | 5 | | 5 | | 3 | | 3 | |
|---|---|---|---|---|---|---|---|---|---|---|---|---|
| Temperature °C | 140°C | | 140°C | | 140°C | | 140°C | | 90°C | | 90°C | |
| Water vol.% | 0 | | 0.8 | | 0 | | 0.8 | | 0 | | 0.3 | |
| Residence time hrs | CN | HY | CN | HY | CN | HY | CN | HY | CN | HY | CN | HY |
| 0.0 | 1.43 | 0 | 1.54 | 0 | 2.7 | 0 | 2.64 | 0 | 3.10 | 0 | 3.19 | 0 |
| 0.16 | 0.53 | | 0.24 | | 1.51 | | 0.46 | | 0.40 | | 0.27 | |
| 0.33 | 0.37 | | 0.12 | | 1.19 | | 0.15 | | 0.37 | | 0.37 | |
| 0.5 | 0.26 | | 0.07 | | 0.83 | | 0.03 | | 0.35 | 0.17 | 0.15 | 0.0 |
| 0.66 | 0.26 | 0.36 | 0.06 | 0 | 0.63 | 0.2 | 0.03 | 0 | | | | |

The catalysts used were as follows

| Catalyst 1 | Supported Reduced Nickel Commercially available as CALSICAT X 475SR |
|---|---|
| Catalyst 2 | Supported Reduced Nickel Commercially available as GIRDLER G 49SR |
| Catalyst 3 | Supported Reduced Nickel Commercially available as HARSHAW 3266E 1/16 |
| Catalyst 4 | Sulphided Nickel/Molybdenum Catalyst Commercially available as KETJEN KF 840 |
| Catalyst 5 | Sulphided Nickel/Molybdenum Catalyst Commercially available as PROCATALYSE LD 145 |

CN denotes the carbonyl number of the finished alcohol as measured by the 2-4 dinitrophenylhydrazine method as expressed in mg KOH/gram.

HY is the wt% heavies (products above $C_{10}+$) in the finished alcohol as determined by gas chromatography.

Example 2

The example illustrates the effect of sulphur on the oxo product hydrogenation catalyst, the conditions are the same as Example 1 except that after decobalting the oxo product was passed to a hydrogenation reactor of four tubes in series equipped with intermediate sample points and no distillation and hydrofinishing were employed. The catalyst was cuprous-chrome (G22RS from Girdler), pressure 55 Megapascals, temperature 180°C, space velocity 1.5 volume of oxo product per volume of catalyst per hour, hydrogen 100 normal volumes per volume of oxo product. After 500 hours of operations the following results were obtained.

| Residence Time in Hours | Carbonyl Number mg KOH/g |
|---|---|
| 0 | 88.0 |
| 0.25 | 22.0 |
| 0.50 | 6.7 |
| 0.75 | 3.5 |
| 1.0 | 1.5 |

The operation was then continued but using an oxo product obtained from a feed to oxonation containing sulphur such that the feed to hydrogenation contained 30 ppm sulphur. After 5 days the following results were obtained while maintaining the hydrogenation operating conditions.

| Residence Time in Hours | Carbonyl Number mg KOH/g |
|---|---|
| 0 | 86.0 |
| 0.25 | 67.0 |
| 0.50 | 40.2 |
| 0.75 | 20.0 |
| 1.0 | 8.5 |

Example 3

This Example illustrates the effect of sulphur on the hydrofinishing nickel catalysts, the conditions used were the same as example 1 except the catalyst was a supported reduced nickel catalyst of G134RS from Girdler (Sud-Chemie), and the temperature in hydrogenation was 80°. After 350 hours the following results were obtained.

| Reduced Time in Hours | Carbonyl Number mg KOH/g |
|---|---|
| 0 | 2.95 |
| 0.16 | 0.42 |
| 0.33 | 0.10 |
| 0.50 | 0.05 |
| 0.66 | 0.04 |

The operation was continued for 7 days except that 50 ppm of sulphur were added to the alcohol and the following results were obtained with and without water injected

| Residence Time in Hours | Carbonyl Number mg KOH/g (1) | Carbonyl Number mg KOH/g (2) |
|---|---|---|
| 0 | 3.02 | 3.02 |
| 0.16 | 2.56 | 2.02 |
| 0.33 | 2.10 | 1.20 |
| 0.50 | 1.98 | 0.80 |
| 0.66 | 1.85 | 0.36 |

(1) No water injected
(2) 1 vol % water injected

## Example 4

Example 2 was repeated using a nickel molybdenum sulphided catalyst (KF 840 from Ketjen) and the feed to oxo contained 55 ppm of sulphur to give 45 ppm in the feed to hydrogenation. The following results were obtained.

| Catalyst Life (Days) | 1 | 20 |
|---|---|---|
| Residence Time in Hours | Carbonyl Number mg KOH/g | Carbonyl Number mg KOH/g |
| 0 | 79.37 | 82.3 |
| 0.25 | 3.18 | 3.00 |
| 0.50 | 1.08 | 1.20 |
| 0.75 | 0.89 | 0.80 |
| 1.0 | 0.70 | 0.65 |

Showing no degradation after 20 days operation.

After 30 days of catalyst life the operations were switched to hydrofinishing with and without water injection with a temperature of 140°C, and a pressure of 55 Megapascals, the following results were obtained.

| Water (Vol%) | 0 | 1 |
|---|---|---|
| Residence Time in Hours | Carbonyl Number mg KOH/g | Carbonyl Number mg KOH/g |
| 0 | 2.89 | 3.03 |
| 0.16 | 0.56 | 0.36 |
| 0.33 | 0.46 | 0.16 |
| 0.50 | 0.36 | 0.10 |
| 0.67 | 0.30 | 0.07 |

Showing that when using the conditions of the invention the process can be operated effectively with feeds containing sulphur.

7

EP 0 343 819 A1

**Claims**

1. A process for the production of alcohols comprising
(i) hydroformylation of an olefine
(ii) catalytic hydrogenation of the hydroformylation product
(iii) separation of an alcohol containing fraction from the product of catalytic hydrogenation and
(iv) subjecting the alcohol containing fractions to catalytic hydrofinishing
the improvement in which comprises introducing water into the catalytic hydrofinishing stage of the process.

2. A process according to Claim 1 in which from 0.1 to 1.5 volume % of water based on the feed to the catalytic hydrofinishing is introduced.

3. A process according to claim 1 or claim 2 in which the sulphided nickel/molybdenum, nickel/tungsten or cobalt/molybdenum catalyst is used in the catalytic hydrogenation of the hydroformylation product.

4. A process according to any of the preceding claims in which a sulphided nickel/molybdenum, cobalt/molybdenum or nickel/tungsten catalyst is used in the catalytic hydrofinishing.

5. A process for the production of alcohols comprising
(i) hydroformylation of an olefine feed
(ii) catalytic hydrogenation of the hydroformylation product
(iii) separation of an alcohol containing fraction from the product of catalytic hydrogenation and
(iv) subjecting the alcohol containing fractions to catalytic hydrofinishing in which a sulphided nickel/molybdenum, nickel/tungsten or cobalt/molybdenum catalyst is used in the catalytic hydrogenation stage and/or the catalytic hydrofinishing stage.

6. A process according to claim 5 in which the olefine feed fed to the hydroformylation stage contains more than 5 ppm sulphur.

7. A process according to claim 5 or claim 6 in which water is provided in the catalytic hydrofinishing stage.

8. A process according to claim 7 in which from 0.1 to 1.5 volume % of water based on the feed to hydrofinishing stage is provided.

9. A process according to claim 6 or claim 7 in which the water is injected into the feed to the hydrofinishing stage.

10. Alcohol whenever produced by a process according to any of the preceding claims.

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A- 966 139 (BATAAFSCHE PETROLEUM MAATSCHAPPIJ) <br> * Page 1, left-hand column, paragraph 5, paragraph 7 - page 1, right-hand column, paragraph 4; page 2 * <br> --- | 1 | C 07 C 29/16 <br> C 07 C 31/125 |
| A | FR-A-1 023 436 (STANDARD OIL DEVELOPMENT CO.) <br> * Page 3, left-hand column, last paragraph - right-hand column, paragraph 3; figures 1,2; page 6, example 2; pages 6,7; claims A,C,1,2,B1 * <br> --- | 1 | |
| A | FR-A-1 056 031 (STANDARD OIL DEVELOPMENT CO.) <br> * Page 2, left-hand column, paragraph 3; page 7, claims * <br> --- | 1 | |
| A | US-A-2 671 119 (J.K. MERTZWEILLER) <br> * Column 2, lines 25-37; column 8, claims; figures * <br> ----- | | TECHNICAL FIELDS SEARCHED (Int. Cl.4) <br><br> C 07 C 29/00 <br> C 07 C 31/00 |

The present search report has been drawn up for all claims

| Place of search | Date of completion of the search | Examiner |
|---|---|---|
| THE HAGUE | 12-09-1989 | KINZINGER J.M. |